# EUROPEAN PATENT SPECIFICATION

(11) **EP 3 967 250 B1**
(45) Date of publication and mention of the grant of the patent: **07.05.2025**
(21) Application number: 21152545.6
(22) Date of filing: 20.01.2021
(51) Int. Cl.: A61B 17/70, A61B 17/80

(54) **FLEXIBLE BONE FIXATION DEVICE**
FLEXIBLE KNOCHENFIXIERUNGSVORRICHTUNG
DISPOSITIF DE FIXATION D'OS FLEXIBLE

(30) Priority: 15.09.2020 TW 109131625
(43) Date of publication of application: 16.03.2022
(73) Proprietor: Industrial Technology Research Institute, 31040 Hsinchu (TW)
(72) Inventor: JANG, Fang-Jie, 204 Keelung City (TW); TSAI, Pei-I, 300 Hsinchu City (TW); Chen, An-Li, 736 Tainan City (TW); FAN, Wei-Lun, 350 Miaoli County (TW); LIN, Shih-Ping, 805 Kaohsiung City (TW); WEN, Yi-Hung, 300 Hsinchu City (TW); LIN, De-Yau, 722 Tainan City (TW); YANG, Shun-Mao, 302 Hsinchu County (TW); SU, Ying-Hao, 300 Hsinchu City (TW); KO, Huan-Jang, 300 Hsinchu City (TW)
(74) Representative: dompatent von Kreisler Selting Werner - Partnerschaft von Patent- und Rechtsanwälten mbB

(56) References cited:
- WO-A1-2008/033976
- US-A1- 2007 123 883
- US-A1- 2009 069 812
- US-A1- 2009 093 846
- US-B1- 6 206 882

## Description

### Technical Field

The disclosure relates to a fixation device, more particularly to a flexible bone fixation device.

### Background

An orthopedic cast is a shell, frequently made from plaster to stabilize and hold a broken bone in place until healing is confirmed. In some cases, the broken bones are held with special implants, such as plates and screws that are used for internal fixation. The plates are attached to bone with screws to act like internal splints that hold the broken pieces of bone together. The plates and screws are compatible with the body and rarely cause an allergic reaction, and, in some cases, the plates are inserted into the fracture to make it stronger and speed up healing, thus, it is not always necessary to remove the implant after healing, and which can avoid the risk of additional surgery.

As such, deep implants will be in place in the body for a long time. However, although the conventional implants can support and hold the broken bones, they cannot restore the normal range of motion after the fracture has finished healing. That is, the conventional internal fixations fail to restore the range of the bone's motion.

US 2009/069812 A1 discloses a rib fixation device with an intramedullary nail connected to a generally U-shaped plate.

US 6,206,882 B1 is directed to elongated bone plate with flexure slots terminating at locations spaced from one another and bores extended for receiving bone fastener.

### SUMMARY

The subject matter of the invention is defined by independent claim 1 while preferred embodiments are set forth in the dependent claims.

Accordingly, the present disclosure provides a flexible bone fixation device that is able to provide dynamic support needed for the biomechanics of the applied part so as to improve the restoration after surgery.

One embodiment of the disclosure provides a flexible bone fixation device including two first fixation parts and a first flexible spanning part, connected between the two first fixation parts. The first flexible spanning part has a plurality of first cuts spaced apart by one another.

According to the flexible bone fixation device as discussed in the above embodiments of the disclosure, the first cuts enable the flexibility and resilience of the first flexible spanning part, thus the cooperation of the first fixation parts and the first flexible spanning part allows the flexible bone fixation device to provide a certain degree of stiffness in a specific direction to offer the required support and hold for the applied part while responding to the motion of the applied part. As such, the flexible bone fixation device can provide sufficient dynamic support to improve the restoration of the applied part.

### BRIEF DESCRIPTION OF THE DRAWINGS

The present disclosure will become better understood from the detailed description given hereinbelow and the accompanying drawings which are given by way of illustration only and thus are not intending to limit the present disclosure and wherein:
FIG. 1 is a perspective view of a flexible bone fixation device according to one embodiment of the disclosure;
FIG. 2 shows an example of using the flexible bone fixation device in FIG. 1;
FIG. 3 is a perspective view of a flexible bone fixation device according to an embodiment not covered by the claimed invention; and
FIG. 4 shows an example of using the flexible bone fixation device in FIG. 3.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details.

The following embodiments will be described with reference to the drawings. For the purpose of clear illustration, some conventional elements and components may be illustrated in a simple and clear manner. Some of the features in the drawings may be slightly exaggerated or illustrated in a larger proportion for the ease of viewing but are not intended to limit the disclosure. In addition, for the same reason, some of the elements or components in the drawings may be illustrated in dotted lines.

Herein, the terms, such as "end", "part", "portion", "area", may be used to refer to specific features of or between elements or components but are not intended to limit the elements and components. In addition, the terms, such as "substantially" and "approximately", as used herein may mean a reasonable amount of deviation of the described term such that the end result is not significantly changed.

Further, unless explicitly stated, the term "at least one" as used herein may mean that the quantity of the described element or component is one or larger than one but does not necessarily mean that the quantity is only one. The term "and/or" may be used herein to indicate that either or both of two stated possibilities.

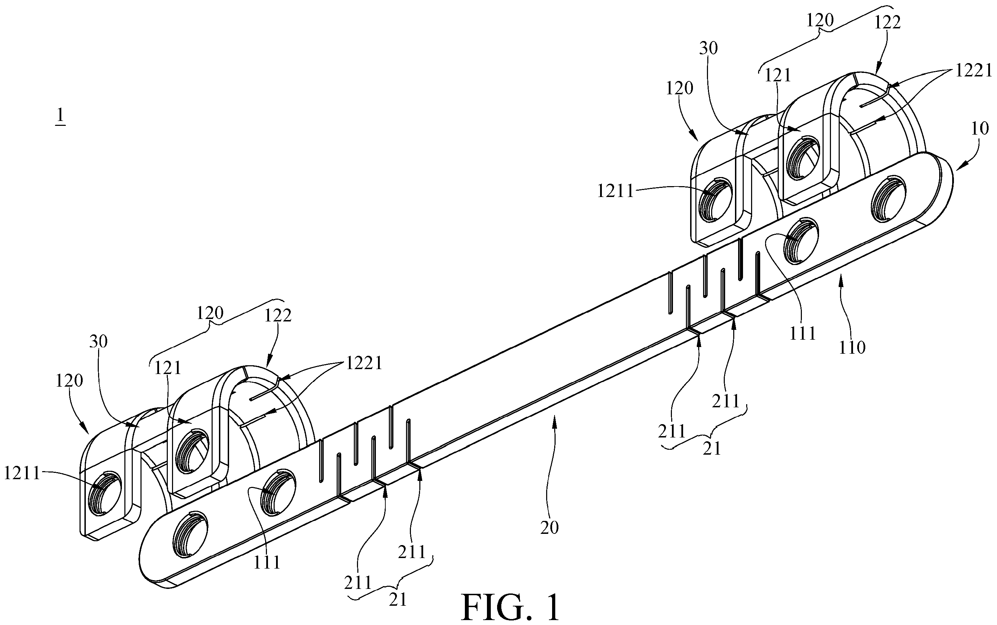

Firstly, referring to FIGS. 1-2, one embodiment of the disclosure provides a flexible bone fixation device 1, the flexible bone fixation device 1 is applicable to ribs 9 (as shown) or other bones in similar shape that can be referred as "applied part". The applied part is defined as the part of a patient that has an indication for the use of the flexible bone fixation device 1 and is likely to come into contact with the flexible bone fixation device 1 during normal use. In addition, for the purpose of illustration, the fracture is not shown. The flexible bone fixation device 1 is made of a material that is biocompatible and flexible and has sufficient strength to resist fracture under a certain amount of force and high endurance limit to prevent fatigue failure, such as stainless steel, titanium, and titanium alloys, but the disclosure is not limited thereto.

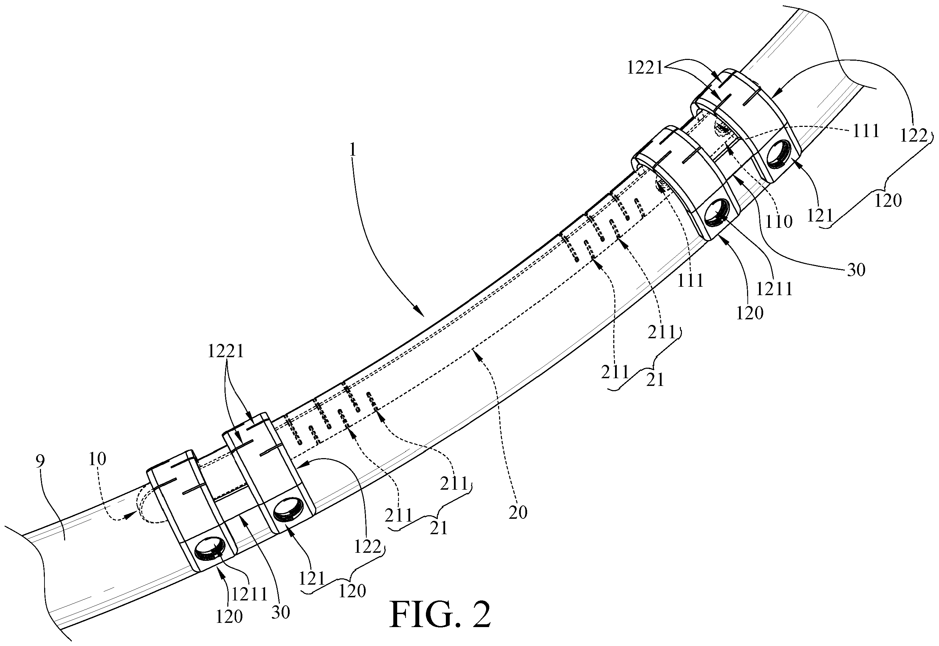

In addition, the flexible bone fixation device 1 has a shape and thickness suitable for being deformed to a desired contour by a surgeon or surgical assistant during or before surgery. In other embodiments, the flexible bone fixation device may have a curvature matching an ergonomics of the applied part without spending extra time on further adjustment (e.g., the curvature shown in FIG. 2) and therefore help reduce surgery time.

Further, in this embodiment, the flexible bone fixation device 1 includes a plurality of first fixation parts 10 and a first flexible spanning part 20 connected between the first fixation parts 10. The first fixation parts 10 are the parts of the flexible bone fixation device 1 for being attached to the applied part. As shown, the first fixation parts 10 can be fixed to the part nearby the fracture of the applied part (i.e., the rib 9). The first flexible spanning part 20 not only can provide support for the applied part but also can have flexibility and resilience that are responsive to the motion of the human body. In this embodiment, the first fixation parts 10 and the first flexible spanning part 20 are, but not limited to, integrally formed as a single piece.

Furthermore, the first fixation parts 10 each include a first fixation portion 110. The first flexible spanning part 20 is connected between the first fixation portions 110 of the first fixation parts 11. The first flexible spanning part 20 and the first fixation portions 110 are, but not limited to, integrally formed as a single piece in a form of a plate. In this embodiment, the first fixation portions 110 each include a plurality of first screw holes 111, the first screw holes 111 penetrate through the first fixation portion 110 and are suitable for the insertion of bone screws into the applied part.

The first flexible spanning part 20 can provide better flexibility and resilience compared to the first fixation portions 110. In detail, the first flexible spanning part 20 may include a plurality of cut arrangements 21 spaced apart from each other and arranged along the first flexible spanning part 20, each of the cut arrangements 21 has a plurality of first cuts 211. As shown, the first cuts 211 extend inward from two opposite edges of the first flexible spanning part 20 and are arranged in an alternate manner so that part of the first flexible spanning part 20 is in a serpentine shape.

In more detail, in this embodiment, a spacing distance between the cut arrangements 21 is substantially larger than a spacing distance between the adjacent first cuts 211 in each cut arrangement 21. The term "adjacent" used herein especially refers to the situation that the two identical elements or components have no other identical elements or components inbetween. As shown, any two of the cut arrangements 21 located adjacent to each other are spaced apart by a spacing distance larger than a spacing distance between any two of the first cuts 211 of each cut arrangement 21 located adjacent to each other. In other words, to all of the first cuts 211 on the first flexible spanning part 20, at least one pair of adjacent first cuts 211 have a spacing distance larger than that of other pairs of adjacent first cuts 211. In addition, in some other embodiments, the first cuts 211 in the same cut arrangement 21 can be spaced apart by a fixed distance, but the fixed distance is at least smaller than the distance between the adjacent cut arrangements 21.

In addition, each of the first cuts 211 has a width ranging between 0.2 millimeters and 2 millimeters, every two of the first cuts 211 located adjacent to each other are spaced apart by a spacing distance ranging between 0.5 millimeters and 5 millimeters, and each of the first cuts 211 has a length or depth ranging between 10% and 90% of a width of the first flexible spanning part 20. The term "width" used herein is measured along a direction substantially perpendicular to the long side of the first flexible spanning part 20.

In such an arrangement, the first flexible spanning part 20 is elastically deformable along specific directions. In one embodiment, the first flexible spanning part 20 is allowed to be elastically deformed with an angle of curvature of ±60°. This makes the flexible bone fixation device 1 able to match the contour of the bone and facilitate the surgery, meanwhile, the first flexible spanning part 20 can move along with the motion of the human body while providing a certain degree of stiffness in specific directions to offer the required support and hold for the bone. That is, the arrangement of the first cuts 211 enables the dynamic support required by the biomechanics of the applied part and therefore can improve the restoration.

Note that the quantity and arrangement of the cut arrangements 21 and the first cuts 211 can be modified according to actual requirements, such as the required flexibility and stiffness or the curvature of the applied part. For example, in other embodiments, some of the first cuts surround the first flexible spanning part and do not cut through the first flexible spanning part. In yet another embodiment, the first cuts may have different lengths. In yet still another embodiment, the closed end of some of the first cuts may have a circular shape having a diameter ranging between 0.5 millimeters and 2 millimeters, in such a case, the ratio of the width of the first cut to the diameter of the circular closed end ranges between 0.5 and 1.

In addition, according to analysis result, when the width of the first cut 211 ranges between 0.2 millimeters and 2 millimeters, the spacing distance between every two adjacent first cuts 211 ranges between 1 millimeter and 5 millimeters, and the thickness of the first flexible spanning part 20 ranges between 1.5 millimeters and 2 millimeters, the flexible bone fixation device 1 can meet the requirements of providing sufficient stiffness and flexibility for ribs.

Further, the quantity and proportion of the first flexible spanning part 20 and the first fixation portion 110 can be modified according to actual requirements. For example, in other embodiments, the length and quantity of the first flexible spanning part and the first fixation part can be adjusted according to various actual requirements.

In addition, in this embodiment, the first fixation parts 10 each include a plurality of hook 120. The hook 120 is connected to the first fixation portion 110 and integrally formed with the first fixation portion 110, but the disclosure is not limited thereto. In other embodiments, the hook 120 may be fixed to the first fixation portion 110 using any suitable fixation means or manner.

In detail, in this embodiment, the hooks 120 each include a second fixation portion 121 and a flexible curved part 122, the second fixation portion 121 is the part of the hook 120 for being fixed to the bone, the flexible curved part 122 is in C shape and connected between the second fixation portion 121 and the first fixation portion 110 so as to keep the second fixation portion 121 and the first fixation portion 110 spacing apart by a proper distance. As shown, the hook 120 is in a shape suitable for being placed on the rib.

In more detail, in this embodiment, the hook 120 includes a plurality of second screw holes 1211 and a plurality of second cuts 1221, the second screw holes 1211 are respectively arranged at the second fixation portions 121 of the hook 120, the second screw holes 1211 penetrate through the second fixation portions 121 and respectively correspond to the first screw holes 111 of the first fixation portions 110, suitable for the insertion of bone screws into the applied part. The second cuts 1221 are arranged at the flexible curved parts 122, and the second cuts 1221 have the same or similar configuration and arrangement to that of the first cuts 211. Thus, similarly, the flexible curved part 122 enables the dynamic support required by the biomechanics of the applied part.

In addition, as shown, in this embodiment, the flexible bone fixation device 1 further includes a plurality of bridge parts 30, the bridge parts 30 are connected between the second fixation portions 121 of the adjacent hooks 120 so as to provide support to the adjacent hooks 120.

As discussed, as shown in FIG. 2, before or during surgery, a surgeon or surgical assistant can adjust the shape of the first flexible spanning part 20 to match the contour of the applied part (e.g., the rib 9), then temporarily place the flexible bone fixation device 1 on the rib 9 by hooking the hooks 120 onto the rib 9, and then can insert any suitable medical screws into the first screw holes 111 of the first fixation parts 10 and the second screw holes 1211 so as to firmly fix two opposite sides of the flexible bone fixation device 1 to the rib 9, finishing the installation of the flexible bone fixation device 1. During the above processes, since the first cuts 211 allow the first flexible spanning part 20 to be elastically deformed in specific directions, which not only facilitates the placement of the flexible bone fixation device 1 but also can provide dynamic support required by the motion of the human body.

Note that the hooks 120 of first fixation part 10 are optional; in some other embodiments, the flexible bone fixation device may omit the aforementioned hooks, and the first fixation portions of the first fixation part are fixed in position simply using medical screws.

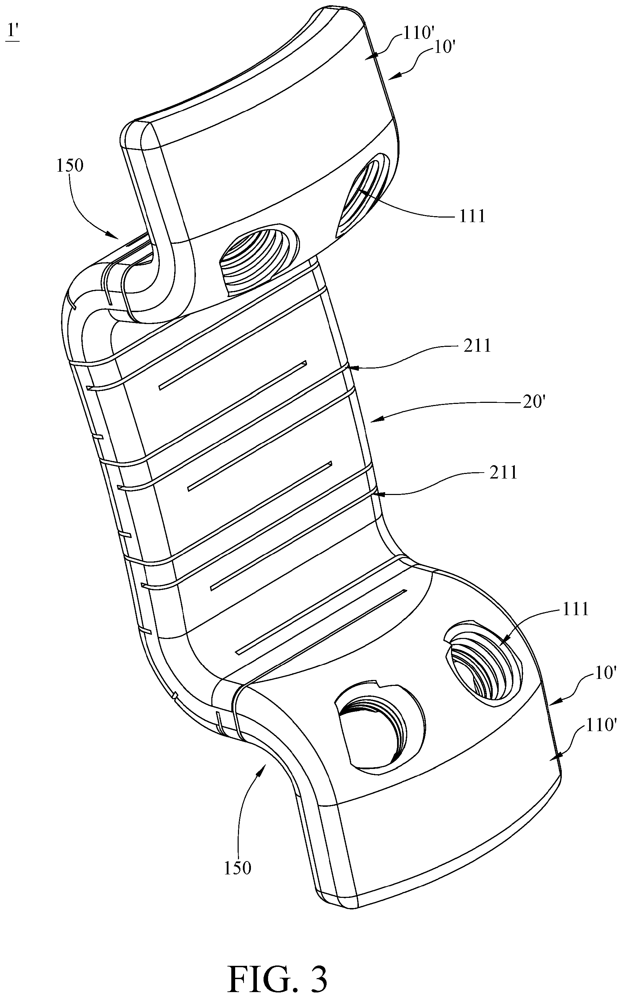

The above embodiment is an exemplary flexible bone fixation device for the fixation of rib, but the disclosure is not limited thereto. For example, please refer to FIGS. 3-4, an embodiment not covered by the claimed invention provides a flexible bone fixation device 1' suitable for the fixation of another part of human body. However, for the purpose of simple illustration, only the differences between the previous and following embodiment will be described in detail, the same or similar part of them will be understood with reference to the previous paragraphs so are not repeated.

As shown, the flexible bone fixation device 1' includes a plurality of first fixation parts 10' and a first flexible spanning part 20' connected between the first fixation parts 10', where the first flexible spanning part 20' also can provide dynamic support needed for the biomechanics of the applied part.

In specific, the first fixation portions 110' of the first fixation part 10' are configured to be fixed to bone and to provide support to the bone, and the first flexible spanning part 20' has first cuts 211' having similar configuration and arrangement to that of the first cuts 211 of the previous embodiment. Thus, the first flexible spanning part 20' can provide a certain degree of stiffness in a specific direction to offer the required support and hold for the applied part while having the flexibility that can move along with the motion of the human body.

In addition, there are two concaves 150 formed between the first flexible spanning part 20' and the first fixation portions 110', the concaves 150 are configured to cover and partially match the contour of the applied part so as to help the flexible bone fixation device 1' fit the bone.

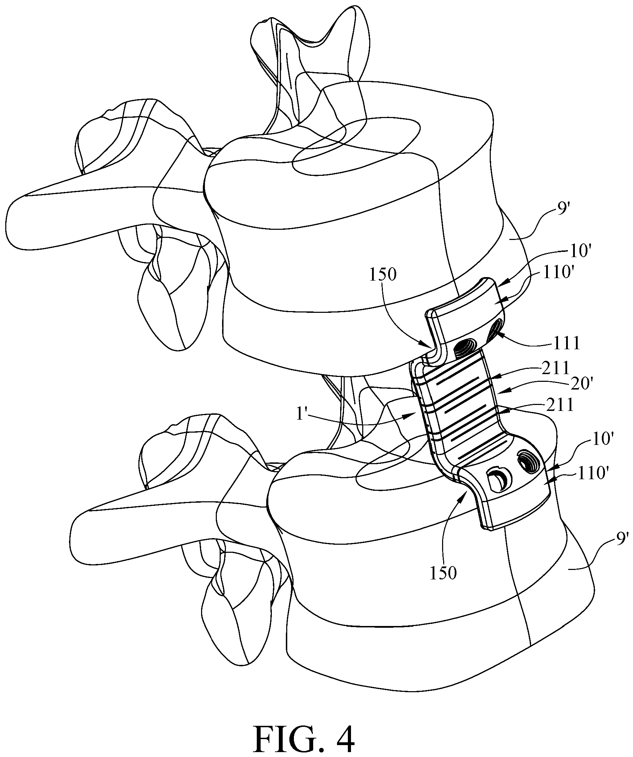

As such, as shown in FIG. 4, the flexible bone fixation device 1' is suitable to be placed in the space between two vertebrae 9', herein, part of each vertebra 9' can rest on the concave 150 and be covered by the concave 150, such that the flexible bone fixation device 1' can firmly attach the vertebrae 9', then the flexible bone fixation device 1' can be securely fixed to the vertebrae 9' by screwing any suitable medical screws into the first screw holes 111 of the first fixation portion 110'. Since the first cuts 211' allow the flexible bone fixation device 1' to be elastically deformed in specific directions, the flexible bone fixation device 1' can provide a certain degree of stiffness in a specific direction to offer the required support and hold for the vertebrae 9' while having the flexibility that can move along with the motion of the vertebrae 9'. Therefore, the flexible bone fixation device 1' can be served as a replacement for the vertebra removed from the vertebrae 9'.

According to the flexible bone fixation device as discussed in the above embodiments of the disclosure, the first cuts enable the flexibility and resilience of the first flexible spanning part, thus the cooperation of the first fixation parts and the first flexible spanning part allows the flexible bone fixation device to provide a certain degree of stiffness in a specific direction to offer the required support and hold for the applied part while responding to the motion of the applied part. As such, the flexible bone fixation device can provide sufficient dynamic support to improve the restoration of the applied part.

It will be apparent to those skilled in the art that various modifications and variations can be made to the present disclosure. It is intended that the specification and examples be considered as exemplary embodiments only, with a scope of the disclosure being indicated by the following claims.

## Claims

1. A flexible bone fixation device (1), comprising:
two first fixation parts (10); and
a first flexible spanning part (20), connected between the two first fixation parts;
wherein the first flexible spanning part has a plurality of first cuts (211),
the plurality of first cuts extend inward from two opposite edges of the first flexible spanning part and are arranged in an alternate manner so that part of the first flexible spanning part is in a serpentine shape, the plurality of first cuts are spaced offset from one another and situated in and along opposing lateral sides formed by the opposite edges of the first flexible spanning part,
**characterized in that** the plurality of first cuts are divided into a plurality of cut arrangements (21), any two of the plurality of cut arrangements located adjacent to each other are spaced apart by a spacing distance larger than a spacing distance between any two of the first cuts of each of the plurality of cut arrangements located adjacent to each other.

2. The flexible bone fixation device according to claim 1, wherein the first fixation parts each comprise a first fixation portion (110) having at least one first screw hole (111).

3. The flexible bone fixation device according to claim 2, wherein the first fixation parts each further comprise at least one hook (120) comprising a second fixation portion (121) and a flexible curved part (122) which is connected between the first fixation portion and the second fixation portion so that the second fixation portion corresponds to the first fixation portion.

4. The flexible bone fixation device according to claim 3, wherein the flexible curved part has a plurality of second cuts (1221) spaced apart from one another.

5. The flexible bone fixation device according to claim 3, wherein the second fixation portions of the first fixation parts each have a second screw hole (1211), and the second screw holes of the second fixation portions respectively correspond to the first screw holes of the first fixation parts.

6. The flexible bone fixation device according to claim 3, further comprising a plurality of bridge parts (30) respectively connected between the second fixation portions of the hooks located adjacent to each other.

7. The flexible bone fixation device according to claim 1, wherein the plurality of first cuts each have a width ranging between 0.2 millimeters and 2 millimeters.

8. The flexible bone fixation device according to claim 1, wherein every two of the plurality of first cuts located adjacent to each other are spaced apart by a distance ranging between 0.5 millimeters and 5 millimeters.

9. The flexible bone fixation device according to claim 1, wherein the plurality of first cuts each have a depth ranging between 10% and 90% of a width of the first flexible spanning part.

10. The flexible bone fixation device according to claim 1, wherein the first flexible spanning part and the first fixation parts have a plurality of concaves (150) therebetween for fitting a bone surface.

## Patentansprüche

1. Flexible Knochenfixierungsvorrichtung (1), die aufweist:
zwei erste Fixierungsteile (10); und
ein erstes flexibles Überspannungsteil (20), das zwischen den beiden ersten Fixierungsteilen verbunden ist;
wobei das erste flexible Überspannungsteil eine Vielzahl von ersten Schnitten (211) aufweist, wobei sich die Vielzahl von ersten Schnitten von den beiden entgegengesetzten Rändern des ersten flexiblen Überspannungsteils nach innen erstrecken und abwechselnd angeordnet sind, so dass ein Teil des ersten flexiblen Überspannungsteils schlangenförmig ist, wobei die Vielzahl von ersten Schnitten versetzt voneinander beabstandet sind und in und entlang entgegengesetzten lateralen Seiten platziert sind, die durch die entgegengesetzten Ränder des ersten flexiblen Überspannungsteils gebildet werden,
**dadurch gekennzeichnet, dass**
die Vielzahl von ersten Schnitten in eine Vielzahl von Schnittanordnungen (21) geteilt sind, wobei zwei beliebige der Vielzahl von Schnittanordnungen, die aneinander angrenzen, um einen Abstand voneinander bestandet sind, der größer ist als ein Abstand zwischen zwei beliebigen der ersten Schnitte jeder der Vielzahl von Schnittanordnungen, die aneinander angrenzen.

2. Flexible Knochenfixierungsvorrichtung nach Anspruch 1, wobei die ersten Fixierungsteile jeweils einen ersten Fixierungsabschnitt (110) mit mindestens einem ersten Schraubenloch (111) aufweisen.

3. Flexible Knochenfixierungsvorrichtung nach Anspruch 2, wobei die ersten Fixierungsteile jeweils ferner mindestens einen Haken (120) aufweisen, der einen zweiten Fixierungsabschnitt (121) und einen flexiblen gebogenen Teil (122) aufweist, der zwischen dem ersten Fixierungsabschnitt und dem zweiten Fixierungsabschnitt verbunden ist, so dass der zweite Fixierungsabschnitt dem ersten Fixierungsabschnitt entspricht.

4. Flexible Knochenfixierungsvorrichtung nach Anspruch 3, wobei der flexible gebogene Teil eine Vielzahl von zweiten Schnitten (1221) aufweist, die voneinander beabstandet sind.

5. Flexible Knochenfixierungsvorrichtung nach Anspruch 3, wobei die zweiten Fixierungsabschnitte der ersten Fixierungsteile jeweils ein zweites Schraubenloch (1211) aufweisen, und wobei die zweiten Schraubenlöcher der zweiten Fixierungsabschnitte jeweils den ersten Schraubenlöchern der ersten Fixierungsteile entsprechen.

6. Flexible Knochenfixierungsvorrichtung nach Anspruch 3, die ferner eine Vielzahl von Brückenteilen (30) aufweist, die jeweils zwischen den zweiten Fixierungsabschnitten der Haken verbunden sind, die aneinander angrenzen.

7. Flexible Knochenfixierungsvorrichtung nach Anspruch 1, wobei die Vielzahl von ersten Schnitten jeweils eine Breite im Bereich zwischen 0,2 Millimetern und 2 Millimetern aufweisen.

8. Flexible Knochenfixierungsvorrichtung nach Anspruch 1, wobei jeweils zwei der Vielzahl von ersten Schnitten, die aneinander angrenzen, um einen Abstand voneinander beabstandet sind, der in einem Bereich zwischen 0,5 Millimetern und 5 Millimetern liegt.

9. Flexible Knochenfixierungsvorrichtung nach Anspruch 1, wobei die Vielzahl von ersten Schnitten jeweils eine Tiefe aufweisen, die in einem Bereich zwischen 10 % und 90 % einer Breite des ersten flexiblen Überspannungsteils liegt.

10. Flexible Knochenfixierungsvorrichtung nach Anspruch 1, wobei das erste flexible Überspannungsteil und die ersten Fixierungsteile dazwischen eine Vielzahl von Rundhöhlungen (150) zur Einpassung einer Knochenoberfläche aufweisen.

## Revendications

1. Dispositif de fixation d'os flexible (1) comprenant :
deux premières pièces de fixation (10) et
une première pièce flexible formant pont (20) attachée entre les deux premières pièces de fixation,
la première pièce flexible formant pont ayant une pluralité de premières incisions (211),
la pluralité de premières incisions s'étendant vers l'intérieur à partir de deux bords opposés de la première pièce flexible formant pont et étant agencées en alternance de façon qu'une partie de la pièce flexible formant pont soit en forme de serpentins, la pluralité de premières incisions étant espacées les unes des autres et disposées sur des, et le long de, côtés latéraux opposés formés par les bords opposés de la pièce flexible formant pont,
**caractérisé en ce que** la pluralité de premières incisions sont reparties en une pluralité d'agencements d'incisions (21), deux quelconques de la pluralité d'agencements d'incisions disposés les uns à côté des autres sont espacés d'une distance d'espacement supérieure à une distance d'espacement entre deux quelconques des premières incisions de chacun des agencements d'incisions disposés les uns à côté des autres.

2. Dispositif de fixation d'os flexible selon la revendication 1, les premières parties de fixation comprenant chacune une première partie de fixation (10) ayant au moins un premier trou de vis (111).

3. Dispositif de fixation d'os flexible selon la revendication 2, les premières parties de fixation comprenant chacune en outre au moins un crochet (120) comportant une deuxième partie de fixation (121) et une partie courbée flexible qui est attachée entre la première partie de fixation et la deuxième partie de fixation de façon que la deuxième partie de fixation corresponde à la première partie de fixation.

4. Dispositif de fixation d'os flexible selon la revendication 3, la partie courbée flexible ayant une pluralité de deuxièmes incisions (1221) espacées les unes des autres.

5. Dispositif de fixation d'os flexible selon la revendication 3, les deuxièmes parties de fixation des premières pièces de fixation ayant chacune un deuxième trou de vis (1211) et les deuxièmes trous de vis des deuxièmes parties de fixation correspondant chacun aux premiers trous de vis des premières parties de fixation.

6. Dispositif de fixation d'os flexible selon la revendication 3, comprenant en outre une pluralité de pièces de pont (30) attachées respectivement entre les deuxièmes parties de fixation des crochets disposés les uns à côté des autres.

7. Dispositif de fixation d'os flexible selon la revendication 1, la pluralité de premières incisions ayant chacune une largeur entre 0,2 millimètres et 2 millimètres.

8. Dispositif de fixation d'os flexible selon la revendication 1, deux quelconques de la pluralité de premières incisions disposées les unes à côté des autres étant espacées d'une distance entre 0,5 millimètres et 5 millimètres.

9. Dispositif de fixation d'os flexible selon la revendication 1, la pluralité de premières incisions ayant chacune une profondeur entre 10 % et 90 % d'une largeur de la première pièce flexible formant pont.

10. Dispositif de fixation d'os flexible selon la revendication 1, la première pièce flexible formant pont et les premières pièces de fixation ayant une pluralité de creux (150) entre elles pour aller avec une surface d'os.
